# EUROPEAN PATENT SPECIFICATION

(11) **EP 0 575 410 B1**
(45) Date of publication and mention of the grant of the patent: **19.05.1999**
(21) Application number: 92906372.5
(22) Date of filing: 09.03.1992
(51) Int. Cl.: C12Q 1/02, G01N 33/566, C12Q 1/70, G01N 33/68, C12N 15/73, C12N 15/13, C12N 15/70

(54) **SELECTION OF SPECIFIC PROTEINS BY A BIOLOGICAL METHOD**
AUSWAHL SPEZIFISCHER PROTEINE DURCH EIN BIOLOGISCHES VERFAHREN
SELECTION DE PROTEINS SPECIFIQUES PAR UNE METHODE BIOLOGIQUE

(30) Priority: 07.03.1991 GB 9104801; 20.01.1992 GB 9201127
(43) Date of publication of application: 29.12.1993
(73) Proprietor: BRADBURY, Andrew Raymon Morton, London N7 OEB (GB)
(72) Inventor: BRADBURY, Andrew Raymon Morton, London N7 OEB (GB)
(74) Representative: Perry, Robert Edward
(86) International application number: GB9200419
(87) International publication number: WO9215702

(56) References cited:
- WO-A-91/17271
- WO-A-91/18980
- GB-A- 2 034 717
- SCIENCE. vol. 249, 27 July 1990, LANCASTER, PA US pages 386 - 390; J.K.SCOTT ET AL.: 'Searching for peptide ligands with an epitope library'
- GENE. vol. 70, 30 October 1988, AMSTERDAM NL pages 181 - 189; A.CHARBIT ET AL.: 'Versatility of a vector for expressing foreign polypeptides at the surface of Gram-negative bacteria' cited in the application
- NATURE. vol. 352, 15 August 1991, LONDON GB pages 624 - 628; T.CLACKSON ET AL.: 'Making antibody fragment using phage display libraries'
- BIO/TECHNOLOGY, VOL.11, p.1565-1569, (1993)

## Description

### Field of the Invention

The present invention relates to a biological method to select useful molecules from libraries of chemical specificities of a protein nature encoded by nucleic acid sequences. In one embodiment, this method relies upon a modification of the normal infectious interaction between bacteriophage and bacterium. The invention utilises the fact that there is a phage receptor for bacteria (hereinafter "PRB") and a bacterial receptor for phage (hereinafter "BRP").

### Background of the Invention

It is known that peptides, proteins and antibody domains may be expressed in bacteria or filamentous phage. Vectors for this purpose include plasmids, phagemids and fd or M13 derivatives.

It is also known to clone antibody variable regions by PCR (polymerase chain reaction) technology from biological tissues or cell lines, or by in vitro methods.

Protein epitopes can be expressed on the outer surface of bacteria by cloning them, in frame, into different sites within outer membrane proteins (OMPs), some of which act as receptors for bacteriophages. That such cloned epitopes, expressed in these systems, can be recognised by antibodies is demonstrated by the fact that they have been used as vaccines; see Charbit et al, Gene (1988) 70:181-189.

The infection of bacteria by bacteriophage involves the interaction of a protein or other molecule (the PRB), usually at one end of the bacteriophage (e.g. J protein in lambda, gene III product in M13 and fd, gene 37 protein in T4 phages, gene 38 protein in T2 and Ox2 phages), with a protein or other molecule (the BRP) found on the surface of the bacteria (e.g. lamB on E. coli for lambda, and a component of the sex pilus on E. coli for M13 and fd, ompC or lipopolysaccharide for T4 phages, ompA for Ox2, and ompF for T2).

The PRBs of different phages usually have a number of domains with different functions, only one of which is responsible for binding to the BRP. In the case of the J protein, for example, it has been shown that the N terminal part of the protein is responsible for assembly of the phage tail, while the C terminal domain is responsible for the host range. Similarly in the gene 37 protein of T4 phages, the BRP-recognising region of the PRB is found at the terminal end. In the case of fd, it is the N terminal part of the gene III protein which is responsible for binding to the F' pilus, while the C terminal has a structural role in virus assembly.

Boulain et al, Mol. Gen. Genet. (1986) 205:339-348, show that, in LamB, the BRP for phage lambda, the expression of epitopes in some sites disrupts the normal phage/bacterial interaction and inhibits infection, whereas other sites allow the expression of epitopes without affecting this interaction. Further, when the normal phage/bacterial interaction is inhibited by the expression of an epitope within the BRP (e.g. in lamB), this inhibition can be overcome by mutations within the PRB (the J protein). As the E. coli strains which these J gene host range mutants can infect is not limited to a unique peptide insertion within lamB, but apply to a number of different ones, it is likely that the normal PRB/BRP interaction has been substituted by an alternative one which is nevertheless found on the same proteins.

In some cases, evolutionary relationships can be seen between similar phages with different host ranges. In these cases, the PRB may retain some homology in the part required for phage assembly, but is usually different in the part responsible for the host range, e.g. lambda and 434, fd and IKe; see, respectively, Fuerst et al, Virol. (1978) 87: 437-458, and Peeters et al, J. Mol. Biol. (1985) 181: 27-39.

In some cases it has been possible to change host range specificity of bacteriophages in a predictable fashion by exchanging the BRP binding domains between related bacteriophages. For example, exchanging the 3' region of the J gene in lambda with that of 434 (a related phage), in the absence of any other changes, gives the recombinant lambda the host range of the 434 phage; see Fuerst et al (1978) supra.

Antibodies against the BRP can inhibit phage infection by interfering with phage binding. This has been shown, for lambda and K10 for monoclonal antibodies against lamB, by Gabay et al, J. Biol. Chem. (1982) 257: 6627-6630.

Methods are available, to produce extremely diverse libraries of peptides; see, for example, Cwirle et al, PNAS USA (1990) 87: 6378-6382, and Scott and Smith, Science (1990) 249:386-390. The value of these libraries is that a large and important part of the epitope universe (this may even be a complete repertoire of specificities), from which individual members can be selected using monoclonal antibodies as selectors, can be encompassed in a few microlitres of solution. These libraries are presently created by amplification of random sequences created by oligonucleotide synthesis, e.g. using PCR.

Marks et al, J. Mol Biol. (1991) 222: 581-597, describe the production of libraries of antibody domains in filamentous phage. The use of living microorganisms to produce the library is useful in that it allows easy amplification, manipulation and storage of the library and the selected antibody once isolated.

The selection of appropriate phage antibodies from a library of such phage antibodies is presently performed by successive rounds of purification on a solid phase support. Phage peptides and antibodies of useful affinity can be selected from a "naive" library (i.e. one from an unimmunised animal) using this method. An important aspect of this finding is that the DNA sequences encoding the phage antibody are physically linked to the antibody; consequently the DNA encoding the phage antibody is co-purified with its binding specificity.

More generally, in the exploitation of such libraries (antibody or peptide), the selection procedure relies on the use of a known molecule (hereinafter "the selector"). The selector is usually a solid-phase selector, i.e. coupled to a solid-phase substrate (e.g. coated plate, magnetic bead, tube or column). It is used to challenge the library to select an element which binds to it (hereinafter "the selected element").

In order to have knowledge of the selector, it must be independently analysed and synthesised or purified, in workable amounts. Both synthesis and purification are generally expensive and inefficient. This is now realised to be a problem that can be overcome.

To date, to derive an antibody, be it in bacterial or myeloma cells, it is necessary to purify antigen in workable amounts, a procedure which is not only inefficient and expensive, but also often unique to each antigen. It is clear that a procedure which is able to derive antibodies against protein antigens utilising only the cloned gene sequences encoding such an antigen would be a breakthrough.

### Summary of the Invention

The present invention is directed to a method for selecting a binding partner for a selector protein from among a library of proteins expressed on the surface of a first transformed microorganism, which comprises screening the library with a second microorganism transformed such that it expresses the selector protein on its surface, wherein one of the first and second microorganisms is a bacterium and the other is a bacteriophage.

One embodiment of the present invention is based on the realisation that the specific molecular natures of the PRB and BRP are less important for infection than the fact that they are able to bind to one another, and that the living nature of a library may also be exploited in the selection procedure, by replacing the interaction of BRP with PRB with that of an interacting antibody/antigen or other peptide (protein)/peptide (protein) pair. The selector does not need to be synthesised or purified; all that is required is a DNA sequence which codes for the selector. DNA sequences, either from cloned genes, libraries or randomly generated, are far more easily manipulated and generated than their corresponding peptide or protein counterparts.

More particularly, embodiments of the present invention utilise the normal productive interaction between bacteria and phage. For example, desired antibody specificities expressed on phage particles (phage antibodies) can be selected and amplified from phage antibody libraries by biological means, without recourse to the experimental manipulation of any protein or peptide intermediates. By modification of the interaction between a bacterial cell and a phage, the normal BRP/PRB interaction, which leads to transfer of genetic information from phage to bacteria, is replaced with an antigen/antibody interaction (or indeed any other pair of proteins or peptides which are able to bind to one another), also leading to transfer of genetic information from phage to bacteria.

The permissivity of the PRB (gene III) in fd to foreign protein insertion is well documented and is being used to select useful molecules using the solid phase selector systems described above, whereas in the lambda system, the permissivity of the BRP (lamB) to foreign protein insertion is well documented and has been used to develop useful antibodies by classical methods. It is clear, therefore, that there are examples of successful manipulation of both BRP and PRB but in different bacterial phage systems. The fact that such manipulations can be performed in diverse heterologous systems indicates that manipulation of both partners of an infecting pair is possible.

Examples of suitable bacteria/phage pairs include: Ike, M13, f1 and fd with E. coli; T even phages with E. coli; RNA phages with E. coli; T odd phages with E. coli; lambdoid phages with E. coli with lambda. A particular interaction is based upon the expression of the antibody within the expression of the antigen within the lamB protein of E. coli.

The interaction may also be, for example, as for any cell virus pair. Examples of such cell/virus interactions include retroviruses with eukaryotic cells and Herpes viruses with eukaryotic cells.

Monoclonal antibodies have been derived which bind to the same part of the BRP as the PRB, suggesting that these could function as PRBs if placed within the correct context.

The interaction may result in lysis of bacteria, in acquisition of a resistance phenotype by bacteria, or in the expression of any new gene in bacteria. It may occur in liquid culture or within or upon solid media.

Further novel procedures described herein reduce the size of antibody variable region domains, to alter the orientation of such domains when forming part of chimeric proteins. Others allow expression of functional antibody domains within the COOH terminal of chimeric proteins.

As an example of such a selective interaction, the proocedure may apply to the selection of an antibody or peptide (from a library of such molecules) interacting with the protein product of a cloned gene, without the need for synthesis or purification of the protein product of that gene. Antibodies against the protein products of genes may thus be derived directly from those genes or proteins. It is also clear that this serves as no more than one application of this procedure, and that the selection of any peptide or protein on the basis of its binding properties can be achieved.

Furthermore, the scheme proposed is not restricted to the selection of an element from a library by a single selector. As described below (scheme 3), the selector may be a library (restricted or general) and, by the confrontation of two such libraries, one expressed on phage, the other on bacteria, it is possible to select for pairs of interacting molecules, as well as for molecules which fail to interact with a given repertoire (scheme 3b). Particularly in this context, the method of the invention allows selection (or discovery) of binding rather than of a binding partner as such.

While the PRB has been used to create libraries of diverse molecules (and to select binding partners from them) in the examples cited above, embodiments of the present invention utilises the fact that the foreign proteins are expressed within the PRB (the use of solid phase selectors does not specifically require expression within the PRB).

The use of living microorganisms to express libraries is useful in that it allows easy amplification, manipulation and storage of the library and the selected peptide or antibody once isolated. Furthermore, the DNA sequences encoding the phage antibody are physically linked to the antibody or peptide, so that the DNA encoding the phage antibody or peptide is co-purified with its binding specificity.

The term antibody as used here refers to ScFv, dAb, Fv, Fab, whole Ab and any derivatives thereof. Phage antibodies refer to any phage particle which expresses an antibody fused to the protein in the phage which acts as the phage receptor for bacteria (PRB).

### Description of the Drawings

In the accompanying drawings:
Figures 1 to 3 each illustrate in a schematic way the starting materials, interactions and products of different procedures that illustrate independent embodiments of the invention, each embodiment being described below in more detail under the corresponding Scheme heading; and
Figure 4 illustrates six alternative J gene/antibody fusion proteins that may be used in the invention.

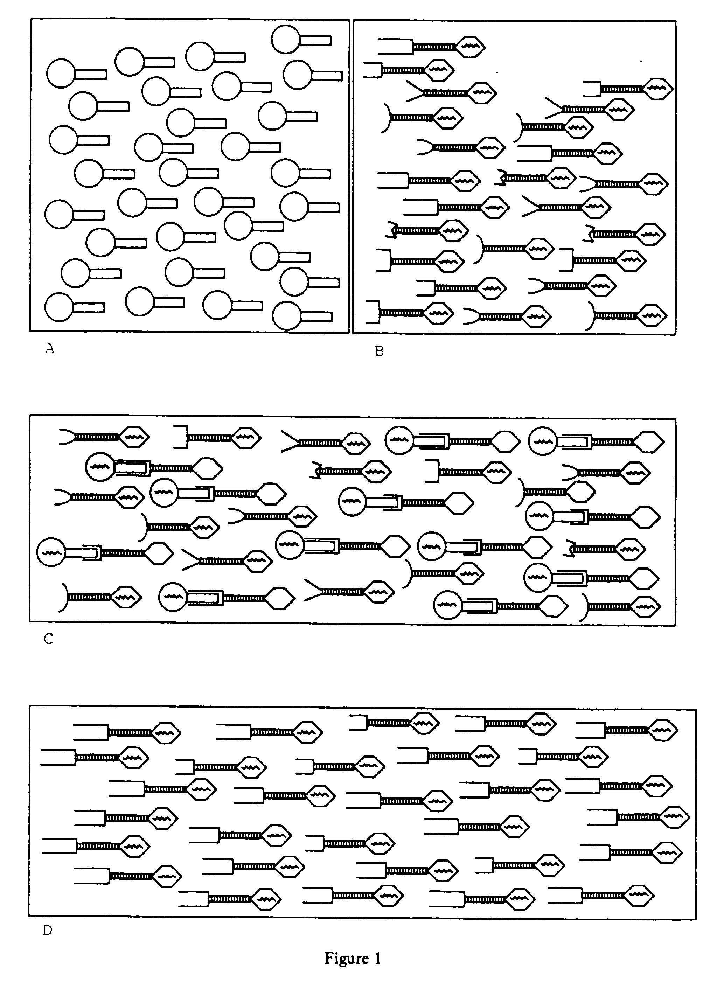

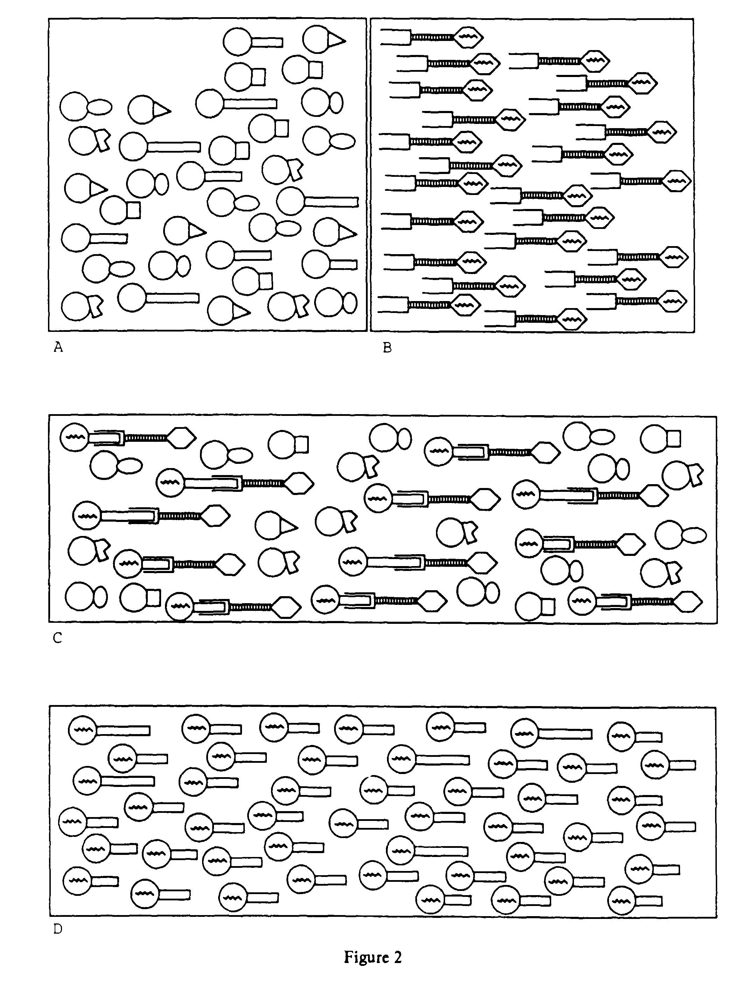

More specifically, Fig. 1A shows bacteria as selector, Fig. 1B phage expressing a library of selectable elements within the PRB, Fig. 1C the binding/non-binding of the phage to the bacteria, and Fig. 1D the result of growing infecting phage under lytic conditions. By contrast, Figs. 2A and 2B show bacteria expressing a library of selectable elements within the BRP and phage as selector, Fig. 2C the binding/non-binding, and Fig. 2D the result of growing (ampicillin-resistant) lysogenic bacteria.

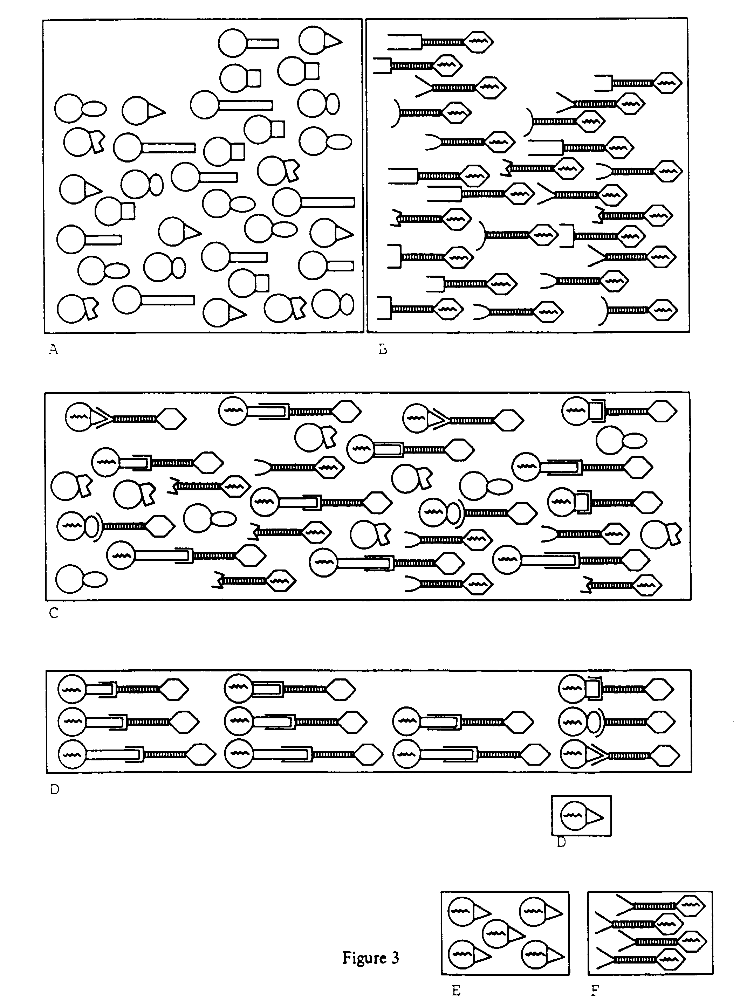

Figs. 3aA and 3bA are each the same as Fig. 2A, Figs. 3aB and 3bB the same as Fig. 1B. Figs 3aC and 3bC each show the confrontation of phage and bacterial libraries. Fig. 3aD shows selected phage/bacterial pairs, Fig. 3aE the selected lysogenic bacterial colony from a single interaction, and Figs. 3aF and 3aG the respective results of amplification of lysogenic bacteria and infectious phage from each individual interaction. Fig. 3bD shows selected phage singles (confrontation under lysogenic or non-infectious conditions, leaving only non-binding or infectious particles). Fig. 3bE shows selected bacterial singles (confrontation under lytic conditions leads to survival and growth of non-infected bacteria).

### Description of the Invention

The four schemes illustrated in the drawings are described, for the purposes of illustrating the invention only, as follows:

In the first scheme (see figure 1), the BRP is replaced by the selector and the PRB with a library of selectable elements encoded by DNA sequences inserted appropriately within the PRB. If the normal phage/bacterial infection mechanism is disrupted in this selection system, then the only way for infection, and hence phage amplification, to occur is by use of the selector/selected element interaction. If lytic phage is used in this scheme, only the selected element, in the form of phage, can be rescued following productive infection. This may be either as plaques after infection in a solid phase, or in supernatants after infection in a liquid phase. The use of non-lytic phage, similarly, will lead to isolation of the phage, either in the supernatant of a liquid phase after infection, or as turbid plaques or resistant colonies (depending upon the nature of the phage used), when plated after infection. In the latter case, the bacteria infected may also be isolated.

In the second scheme (see Fig. 2, using the transfer of antibiotic resistance as an example), the selector is found on the phage surface (within the PRB) and the library of selected elements on the bacterial cell surface (in the BRP). Selection will occur in a fashion similar to that described above, with the difference that one is selecting for a bacterial instead of a phage clone. It is clear that this mechanism will only work where phage infection confers a selectable phenotype (e.g. resistance to ampicillin, tetracycline, chloramphenicol or kanamycin) upon a bacterium, and that bacterium is able to grow as a bacterial clone. Lytic plaques will not occur as there will be no substrate for renewed infection by the phage (since adjacent bacteria will be different and not infectable). In this scheme, both selector and selected element can be recovered after productive infection: selected element by the growth of bacteria from the bacterial clone; selector by the use of appropriate temperature-sensitive phage mutants, or by the natural release of phage into the growth medium during growth of bacteria.

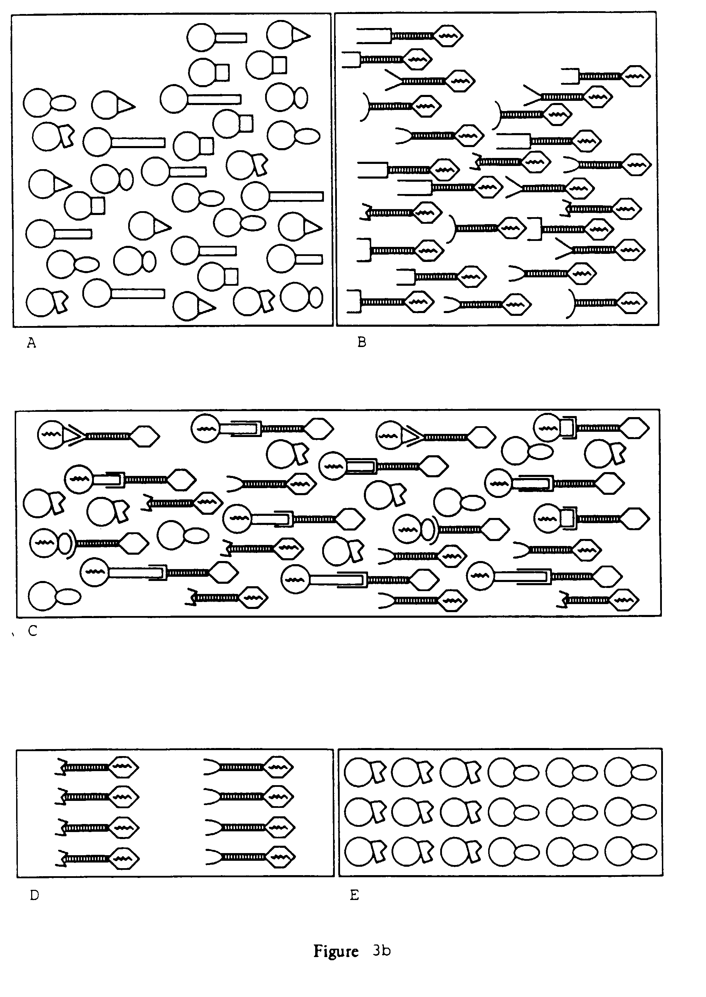

In what is essentially a combination of schemes 1 and 2, two libraries of specificities, one on phage and one on bacteria (within the PRB and BRP, respectively) may be confronted. The successful encounter of phage and bacteria will lead to plaques or colonies which will represent the productive binding of one element of each library (the selection of couples, Fg. 3a). Alternatively, elements not interacting with the library may be selected (the selection of singles, Fig. 3b).

The selector can be soluble, and act to protect the selected element (as expressed as part of the BRP) from interaction with a wild-type lytic phage. In this case, the selector is a soluble protein, the library of selected elements is expressed within the BRP on the surface of bacteria, and bacterial clones surviving after infection with wild-type phage will be expected to bind the selector.

All schemes are applicable to any pair of binding molecules, natural or engineered, and to the selection of elements of any interacting protein pair, of which antibody/antigen pairs are one of many. The demonstration that these schemes are able to work requires the substitution of a normal BRP/PRB pair with another pair of interacting molecules. The ways in which both sides of diverse bacterial/phage system can be manipulated as required for carrying out the method of this invention are described below for a number of different systems. The implementation of these schemes will be illustrated by reference to the selection of either antibodies or antigens (and hence the DNA sequences encoding them), although it should be borne in mind that this is a technique which is applicable to the isolation of the DNA encoding one or both proteins involved in any binding interaction.

### Scheme 1: BRP as selector (Fig. 1)

The interaction between antigen and antibody is used by way of example. It is possible to start with a cloned gene and derive an antibody-binding domain, or alternatively to start with an antibody and derive a DNA sequence which encodes proteins which will bind to that antibody. In the first case, the cloned gene is expressed on the surface of bacteria within the context of the BRP and challenged with a library of antibodies expressed in the phage within the PRB. Only phage expressing an antibody specificity able to bind protein epitopes encoded by the cloned gene will infect the selector bacteria and hence proliferate. In the second case, the expression of the cloned (and unknown) gene in the BRP on the bacterial cell surface is replaced by the expression of a suitable form of cloned antibody. The library expressed in the PRB now consists of either random peptides or suitably selected cDNA sequences (e.g. a library of cDNA sequences derived from a tissue or cell which is known to express the protein recognised by the antibody). In principle, the two cases are identical, and a discussion of the details of one of them is given.

For the selection of an antibody binding to the protein product of a cloned gene, the PRB is replaced with a fusion protein which contains an antibody domain (ScFv, dAb, Fvs, Fabs, or derivatives thereof), as well as the normal PRB (see below for different forms of the fusion protein). The antibody domain expressed in each phage will be different, and hence have a different specificity. The resulting phage library can be propagated on its normal host using the original recognition specificity of the PRB which should not be affected by the incorporation of the antibody (domain). If the normal recognition specificity of the PRB is lost by the incorporation of an antibody domain, the library may be created and propagated by relying on bacterial clones which express antibiotic resistance encoded by a suitable phage. In this case, phage can be isolated by growth of the bacteria (in liquid culture, on solid media, or a combination of both) and harvesting of supernatant, or by induction.

To select an antibody against the antigen X (the protein product of the cloned gene X), a mutant BRP gene, which has within its coding region a DNA sequence encoding the antigen X (or part of it), is created. The antigen X is expressed in a part of the BRP which is required for the normal phage/bacterial infectious interaction. The result is that the protein product of such a fusion gene (BRP-X) will not be permissive for infection by the normal BRP/PRB interaction. If BRP-X is expressed in a BRP (BRP negative) bacterial host, the normal phage/bacterial infection mechanism will be disrupted as the only bacterial receptor for phage present (BRP-X) is unable to bind the normal phage receptor for bacteria. If such a bacterial cell is confronted with a phage antibody library, the only way for infection, and hence phage (and phage antibody) amplification, to occur is if an alternative recognition system exists. The antigen X/anti-X antibody interaction provides such a system and allows amplification of the anti-X phage antibody. Phage antibodies with other specificities will fail to be amplified. Thus, an antibody recognising antigen X can be derived both as protein (as phage antibody) and as a cloned DNA sequence after confrontation of the phage library with the BRP-X-expressing bacterial host.

In the event that the starting library is not complete in its antigen recognition capability, with the result that no antibodies, or ones of low specificity, are obtained, it is a simple matter to immunise mice with the bacterial strain expressing the recombinant BRP-X. The mice (which may be made tolerant to the bacterial host) will mount an immune response, providing a source for a new library enriched for antibody specificities against X. In this case too, it is clear that there is no need to purify the antigen to immunise the mouse, as it is known that BRP-X can act as a good vaccine.

An alternative method to improve (and even modify) phage antibody specificities is by the creation of hierarchical libraries, which involves taking an antibody with a known affinity for an antigen and seeing if this affinity can be changed by keeping one chain (e.g. the heavy chain) fixed and varying the other in the form of a library. This library of antibodies is then screened against the antigen. Once a new antibody has been derived (containing one original chain and a new one found in the library), the process can be repeated, varying the chain which was previously held constant.

The ingredients of this system are: PRB which retains its wild-type specificity (this makes propagation easier, although is not essential) as well as the new antibody specificity which will form part of the antibody library; and two bacterial strains, one wild type to propagate the library, and one mutant strain in which the wild-type BRP domain is substituted by the fusion protein BRP-X, and in which infection can only occur via the interaction of X/anti-X.

The BRP used on BRP-X need not be the same BRP used to propagate the library, and does not even need to be the BRP normally recognised by the PRB used by the virus. It may be a BRP used by another virus which may or may not be related. It is important only that after PRB-(anti-X) has bound to BRP-X that transfer of the phage genetic material passes into the bacteria expressing BRP-X. The use of techniques such as electroporation or low salts, may allow this to occur in cases in which the choice of PRB-(anti-X) and BRP-X allows binding but not infection.

Selection can occur either in liquid phase, in agar or in a combination of liquid followed by agar. In liquid culture, one expects the overgrowth or survival of the phage expressing the desired binding activity. In agar, plaque formation (or the formation of resistant colonies) reflects the successful interaction between phage and bacteria.

The system can, in principle, exploit different bacteriophage/bacterial host interactions, such as the coliphages lambda, M13 (f1, fd), T even phages, T odd phages, RNA phages or even phages of other microorganisms. By way of example, the procedure may be used to engineer this system first in the lambda/lamB system, secondly in the fd/sex pilus system, and thirdly using a mixed system. It is clear that equivalent manipulations may be made in PRB and BRP pairs as described above (T4 gene 37 protein/ompC, T2 gene 38 protein/ompF), and any others, as well as the artificial modification of one of the pairs so that the host range is changed, or the creation or engineering of novel BRP or PRB.

A demonstration that the procedure is able to function can be provided by the creation and use of an antibody of known specificity. Y13.259 is a monoclonal antibody which recognises a 17 amino-acid epitope found within the p21ras molecule. The manner in which the expression of the variable regions of this antibody as ScFv, FAb or Fv as part of the J protein, and the expression of the corresponding epitope within the lamB protein, is as follows:

### Construction of anti-ras (anti-X) lambda antibodies

Strains: DH5αF' was used for all general cloning, Pop6510 for expression of all lamB derivatives, and LE392 for amplification of lambda.

Except where otherwise stated, all methods used are as described in "Molecular Cloning: A Laboratory Manual" (1989) Cold Spring Harbour Laboratory Press. Vector construction: ScFv, FAb or Fv forms of Y13.259 (see Werge et al (1990) FEBS Lett. 274 193-198), under the control of a promoter, are constructed by taking the isolated heavy and light chains and inserting them into vectors which allow expression of the appropriate antibody form. This done by standard cloning techniques, PCR, or PCR-induced ligation techniques. Such vectors are widely available and the important elements include: a bacterial promoter (e.g. LacZ), a ribosome-binding site, an appropriate bacterial leader sequence (e.g. pe1B, ompA), stop codons at the ends of chains, and an appropriate linker sequence between heavy and light chains. For ScFv, such a linker sequence would encode a peptide linker which would connect VH to VL, e.g. [gly₄ser]₃ or the cellulase linker; for Fv, the DNA linker sequence encodes a stop codon at the end of the upstream variable region, a ribosome binding site, and a bacterial leader sequence; for FAb, the variable regions are attached to the CHl and CLl constant regions and separated by linkers similar to those found in Fv. A protein tag (e.g. the myc tag) to detect the cloned antibody form is useful, but is not essential. The order of the chains has been shown not to be important in other antibodies, and would be expected not to be important in this case. Other variations on the normal immunoglobulin architecture are easily envisaged (e.g. ScFv attached to light chain constant regions, or heavy chain constant regions lacking CH1). The particulars are not as important as the creation of a cloned antibody form which is able to bind the peptide described. This can be confirmed by Western Blotting, ELISA, binding to a column or any other technique used to detect antigen/antibody binding. ScFv Y13.259 has been produced and shown to bind its epitope.

Once the specificity is confirmed, the DNA encoding the antibody is cloned in frame to a fragment of the J gene of bacteriophage lambda in a suitable position which had been separately cloned from lambda into a general purpose vector (such as pUCl9 or Bluescript).

One important feature of the J gene antibody fusion constructs is that, by way of example, the BstBI site in J (equivalent to nucleotide 18048 in lambda) is retained within the plasmid construct. This is found towards the 3' end of the gene. The genetic evidence (see Fuerst et al, supra) suggests that it separates the host-determining region from the structural component, and so allows one to manipulate the host-determining region of the J gene in a plasmid rather than in lambda. J gene fusions can therefore be made which are then easily cloned back into lambda using BstBI to join the structural part of the normal J gene found in the lambda donor left arm to the recombinant J genes produced in the plasmid. An XbaI site at the 5' or 3' end of the fusion construct serves to clone the J gene fusion construct back into lambda by providing a site at the 3' end of the J gene.

These sites are convenient, but any other sites which serve similar functions could be used, and even engineered sites (e.g. rare cutting sites such as NotI or SfiI) could be included within the destination lambda vector as well as the J/Ab fusion vector. Sites further 5' of BstBI (e.g. SnaI, KpnI with remaining sites in J removed) could be used instead of BstBI if a greater part of the J gene is found to be required.

These sites can be used for cloning the J/Ab fusion construct into lambda as follows:
1. Purification of arms: lambda left arm cut with BstBI, and lambda right arm cut with XbaI would be separately prepared and purified (on a gel, or by sucrose density centrifugation in the presence of oligonucleotides complementary to the cohesive end of the arm not required). The source of the lambda arms will determine the phenotype of the esulting recombinant phage. The use of temperature sensitive repressor (cI857) will allow the harvesting of phage after lysogeny by temperature induction.
2. Cloning: equimolar amounts of the arms are mixed with equivalent amounts of the J/Ab fusion construct cut with XbaI and BstBI. In the case where the XbaI site is directly downstream of J/Ab, the resultant recombinant lambda clone will retain all essential genes, except that the normal J gene is replaced by the J/Ab fusion gene. If the XbaI site is upstream of J/Ab, the resultant recombinant lambda clone will be identical to that above except that the gene for amp resistance (as well as the other plasmid genes) will be also included. Other plasmids containing other resistance markers (e.g. tetracycline, kanamycin or chloramphenicol) may also be used.
3. Packaging: after in vitro packaging and infection into appropriate cells, plaques can be picked, or if the amp resistance has been retained, amp resistant colonies can be picked after plating on low concentration ampicillin plates (20-30 µg/ml) and growing at 30°C. The use of hfl (high frequency of lysogenisation) 1on protease negative bacterial strains would be expected to increase the frequency of amp resistance colonies and stabilise the fusion protein. Phage can be isolated from the colonies by heat induction (to inactivate the cI857 temperature sensitive repressor) or just by growth of the lysogenic bacteria, since phage are released naturally into the culture medium, even at lowered temperatures. The use of lowered temperatures may be important for the stability of the J/Ab fusion protein, as it is when antibodies (as ScFv or Fab) are grown in bacteria.

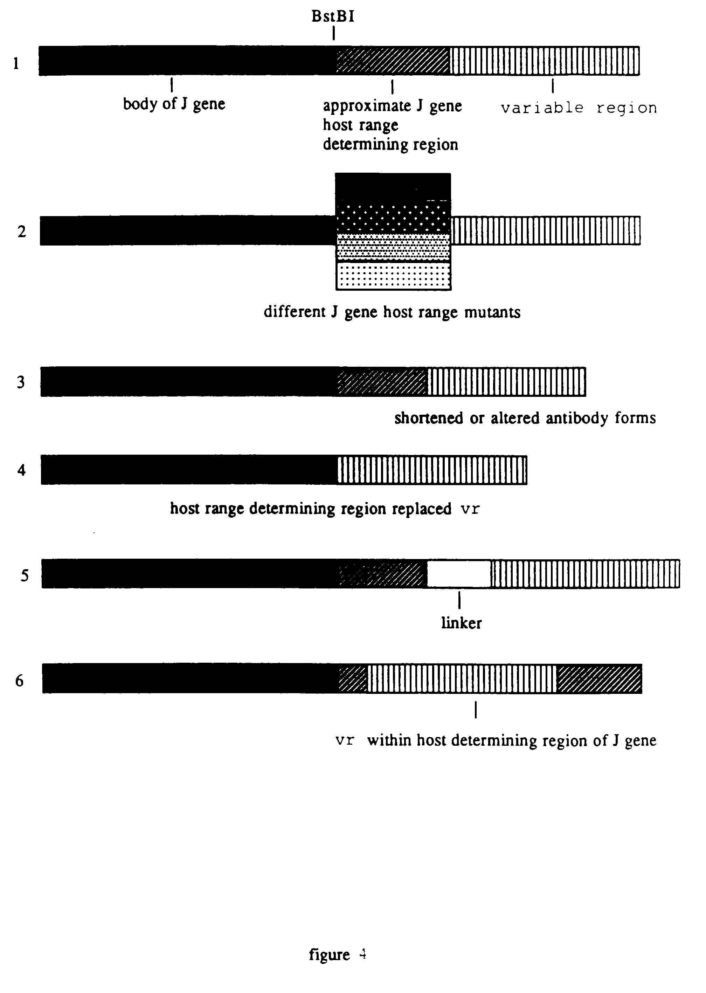

### Alternative forms of lambda antibody constructions (Fig. 4)

Alternative forms of the J/Ab fusion proteins include the following:
1. Instead of using wild-type lambda as the source of the J gene, that part of the J gene responsible for the host range phenotype of strains λho, λhh* and KlO (between bases 18048 and 18890 of lambda) can be used, replacing the wild-type sequence in this position. This may be done using PCR to amplify the corresponding segment of DNA, and standard cloning. These different host range mutants may be differentially sensitive to the presence of the Ab attached to their C terminal end or within the J gene protein.
2. Different antibody forms: deletion of part of the antibody (done using PCR and cloning) may be useful to orientate it in a different direction if there is steric hindrance of J with respect to Ab or vice versa. The removal of a single β strand (e.g. amino-acids L1-14 or H1-13) would be expected to change the orientation of the antibody without affecting its binding specificity. It is clear that any derivative of any antibody or binding molecule can be used. The use of antibody forms which require two separately synthesised chains (FAb, Fv etc) may have one of the two chains synthesised as a soluble intracytoplasmic derivative. This be expected to associate with the other chain covalently linked to J as a fusion protein. In this case bacterial leader sequences would be deleted.
3. The region of the J gene coding for host range specificity (and hence the PRB specificity) may be completely deleted and replaced with any of the antibody forms described. In this case, propagation of the lambda phage occurs in a lysogenic state (after in vitro packaging, infection and selection for antibiotic resistance), but it is expected that successful infection will occur if the BRP (lamB) contains an appropriate antigen (lamB-ras) for the PRB-Ab (J-Y13-259).
4. A linker can also be introduced between the Ab and the J protein. Such a linker may be one found naturally between two protein domains. Argos, J. Mol. Biol. (1990) 211: 943-958, discuss short linkers which can be used, and Takkinen et al, Prot. Eng. (1991) 4 (7): 837-841, describe longer linkers. Alternatively, an artificial linker may be created by the use of contiguous short linkers. A linker may also be found experimentally by the creation of a random linker library between the two domains. This may be done by PCR using appropriate oligonucleotides. This should provide flexibility between the two protein domains without being susceptible to proteolysis, and may overcome steric hindrance.
5. The antibody may be put at a number of different positions within the J gene. One position to insert the antibody is at the 3' end of the J gene (the C terminal end of the protein). Sequencing of that part of the J gene responsible for the host range phenotype of strains λh+, λho, λhh* and KlO (between bases 18048 and 18890 of lambda), may reveal differences between these strains which are responsible for the host range differences. Regions of particular diversity between the strains may be responsible for the host range differences and may better tolerate the insertion of the antibody.
6. In addition to the use of a J/Ab fusion gene, a normal J gene may be provided by a lysogenic heteroimmune phage, or on a plasmid. This allows the creation of a hybrid phage which contained a normal J protein which can be used for propagation in normal hosts, and the recombinant J/Ab protein which can be used for selection. This is similar to the manner in which helper phage provides normal gene III protein in the pHENl system; see Hoogenboom et al, Nuc. Acids. Res. (1991) 19: 4133-4137.
7. Any one, or a combination, of the above modifications can be used (e.g. Ab within the J gene containing a different host range mutation).

### Construction of lamB/ras (BRP-X)

To test the lambda Ab described above, a suitable bacterial host expressing p21ras within lamB on the outer surface of an E. coli which otherwise lacks lamB is required. This may be done by as described by Werge et al, Oncogene (1992) in press, cloning the epitope of p2lras recognised by Y13-259 Ab, itself produced by the hybridisation and phosphorylation of complementary oligonucleotides which encode residues 56-76 of p21ras into the BamH I sites of lamB in vectors AJC352, AJC264 and AJC178; see Charbit et al (1988), supra. The hybridised oligonucleotides have BamH I complementary ends (GATC) at both ends, but only at the 3' end would the BamH I site be recreated. This facilitates mapping. Plasmids containing modified lamB genes may be transfected into pop6510, and bacterial clones containing the p2lras epitope in correct orientations may be tested for their ability to bind anti-LamB serum as well as both native Y13-259, and its derivative forms, by ELISA on intact cells, as well as by western blotting.

### Procedure for the demonstration of infectious lambda antibodies

Phage prepared by amplification in LE392 or other cells (either by lysis or heat induction of lysogeny) may be tested for their ability to bind the p21ras antigen (by e.g. enrichment of phage on a p21ras column) after testing the integrity of the J/Ab fusion protein (by e.g. western blotting), and then for their ability to infect pop6510 cells expressing p21ras by infection of a mid log culture of pop6510 lamB/p21ras and plating of dilutions. The synthesis of the lamB/p21ras may be induced with IPTG in the system described (lamB synthesis is under the control of the tac promoter in AJC plasmids). That the infection occurred by the expected peptide/antibody interaction would be shown by the need for the epitope within lamB to allow infection to occur. That it was possible to recover the Y13-259 lambda antibody from a library would be shown by making various dilutions of the Y13-259 lambda antibody into a library of diverse lambda antibodies created as described below.

### Construction and amplification of a lambda antibody library

Once infection by an interaction different from that of the normal PRB/BRP mechanism has been shown to be feasible, a library of variable regions (VH and VK) may be cloned into the same sites of the J gene. This may be carried out using a similar method to that described by Marks et al (1991), supra except that, after assembly and amplification of the Ab form used, it is cloned into lambda arms cut with appropriate sites (e.g. if the same oligonucleotides were used: SfiI and NotI engineered within the lambda for the purpose).

The recombinant lambda vector encoding the lambda antibody library may be in vitro packaged and infected into LE392 for amplification. The library of lambda antibodies (as phage articles) may be harvested after amplification in liquid (or plate) culture using standard methods (lysis or heat induction).

### Procedure for the selection of lambda antibodies

DNA sequences encoding epitopes (X) from the gene for which an antibody is to be derived are cloned into the lamB gene in frame in the appropriate site using either oligonucleotides, cloned pieces of DNA, or DNA obtained by PCR amplification. The presence of a BamH I cohesive end at each end is required. The recombinant lamB/X clone is transfected into a cell line lacking lamB (pop6510) and the bacterial clone cultured.

Selection of appropriate lambda antibody specificities involves a confrontation between the lambda antibody library and the bacterial clone expressing lamB/X. This involves inoculation of a culture of the bacterial clone culture with an aliquot of the lambda antibody library. After culture, the specific lambda antibody is isolated by plating onto a lawn of the recombinant bacteria (pop6510 lamB/X). Plaques are picked, amplified, and tested for their ability to infect pop6510 lamB/X at a much higher efficiency than the complete library, as well as an inability to infect pop6510 lamB/Y, where Y is a different specificity.

If lambda with a temperature-sensitive mutation in cI (e.g. cI857) is used, and development of plaques occurs at the permissive temperature (32°C), they will be turbid. This allows the growth of lysogenic bacteria which can be induced to produce phage upon heat induction. Alternatively, the use of a selective marker and hfl bacteria (as described above for the cloning procedure of lambda antibodies) can be used to select resistant colonies from which lambda and bacteria can be isolated.

Further tests of the specificity of the lambda antibody (as a lambda phage particle) may involve ELISA, immunoprecipitation and western blotting on pop6510 lamB/X bacterial cells, using appropriate non-X-expressing bacterial strains as controls. Measurement of the affinity of the lambda antibody for its bacterial target can also be done using dilution analysis. Since purified natural antigen is not available, further tests of the lambda antibody specificity on native material are required. This can be done using ELISA, immunoprecipitation or western blotting on appropriate extracts.

The detection of lambda antibody binding can be performed using secondary antibodies against endogenous lambda proteins or tags present on the ScFv/J protein fusion protein.

### Downstream use of lambda antibodies

Lambda antibodies can either be used as phage particles containing antibody specificities, or the heavy and light chain variable regions can be cloned out using standard procedures and inserted into any one of a number of compatible vectors which allow expression of protein in a wide variety of different cellular systems, including prokaryotes, eukaryotes and plants. When cloned into the appropriate vectors the antibody specificity can be coupled to desired effector functions, a review of which is given by Winter et al, Nature (1991) 349: 293-299.

### The fd (fl/M13) sex pilus system

In lambda the PRB and the BRP are well recognised. This is not the case in fd (which in the following discussion will be used interchangably with fl and M13, these being three phages which are essentially identical, but have been used in different studies), where the PRB is well recognised, but the identity of the BRP is less clear. There is little doubt that the BRP resides within the sex pilus, but its molecular nature is less clear (it is possibly f pilin). However, definitive identification of the BRP for fd can be done in a number of standard ways (as described widely for many receptors in the literature). However, scheme 1 may also be used to identify the BRP for fd.

Deletion mutants of gene III previously have revealed that the N terminal region is important in binding to the sex pilus and infection. Under certain conditions, M13 is able to infect F⁻ (lacking the F pilus) bacteria, a region required for this activity having been delimited to the N terminal 99 amino-acids. Deletion mutants of CAT lacking the complete N terminal region (aas 1-196) or just part of it (aas 99-196) should not be able to infect bacteria by the normal route. The latter, but not the former, would be expected to be able to infect F⁻ bacteria, and both would be expected to be able to contain antibodies as a fusion protein with the gene III protein.

Immunisation of mice with either purified pili, or whole F' bacteria, has been shown to yield an antiserum able to inhibit infection of F' bacterial cells with fd. The creation of a library of fd phage antibodies from the spleen of a mouse immunised in this fashion should yield some phage antibody particles which are able to recognise the normal binding site of fd. Some of these phage antibodies would be expected to be able to infect F' cells using as PRB the antibody rather than the normal PRB (which has been deleted). The identification of a phage antibody of this type would be the first step towards the molecular identification of the BRP.

The characterisation of a protein which is recognised by a monoclonal antibody is a common and relatively straightforward procedure, and may be conducted using standard techniques. Once characterised, insertion mutants are created by the insertion of restriction enzyme sites, either in a random fashion as has been done with lamB, or in a more directed fashion using PCR. and prediction of secondary structure (targeting the insertions to those parts of the protein expected to be exposed) is relatively straightforward. The insertion of restriction enzyme sites at strategic positions allows an investigation of the ffect on fd infection, and on the effect of the insertion of extraneous protein. In this manner, a system may be created, in a manner similar to that created for lamB (with the development of appropriate bacterial mutants lacking the BRP, and plasmids expressing the deleted BRP on a plasmid with a selectable marker), with the implementation of the four schemes described above.

### Mixed Systems (Combination of lamB and fd systems):

There is an alternative manner in which a scheme similar to scheme 1 may be operated. This involves cloning of an epitope into lamB, and using the bacteria as a solid phase selector to screen a library of phage antibodies (which may have been derived from a mouse immunised with the same recombinant bacteria). The advantage of the use of recombinant bacteria as a solid phase selector (a "living column") to select phage antibodies is the same as that described for scheme 1 above: an antibody can be derived against the protein product encoded by a DNA sequence just be cloning it into the appropriate vector, without the need to produce or purify the protein.

The library of phage antibodies is precleared with identical bacteria not expressing the recombinant epitope, before using the recombinant bacteria as a selector. After allowing binding to occur, the phage antibody particles are eluted (after washing and resuspending the bacteria) with, e.g. 100 mM trietnylamine or a low pH glycine buffer, the column bacteria which could compete are removed (by centrifugation), and an aliquot of the supernatant containing the phage antibody particles to bacteria able to support the infection of fd phage (TGl, DH5αF', JM101 etc) is added. Theoretically, if the lamB/X fusion protein is expressed in such bacteria, elution and infection could occur in the same bacteria, by just allowing reexpression of the F' pilus. In this case, the individual bacteria expressing the antigen X will not necessarily be infected by the same phage which bind to them. For this reason scheme 3a, as described, is not possible, as individually interacting members will not be co-isolated, although it will be possible to isolate a polyclonal phage stock which will recognise a number of different bacteria expressing different recombinant lamB/X on their surface. It is clear that the use of the living column does not require either of the binding partners to be expressed in the BRP or PRB. Any protein expressed on the surface of either bacteria or phage would be suitable.

### Infection by other means

It is known that fd can infect F⁻ bacteria at an efficiency 10⁻⁶ of that when infecting F' bacteria, suggesting that infection can occur independently of the normal mechanism. A modification of the living column described above could be used to infect F⁻ bacteria. This involves binding of phage antibody to bacteria expressing lamB-X (after preclearing the phage antibody library against bacteria not expressing lamB-X), and then plating directly (with or without treatment such as electroporation, low salt solutions as used to make competent cells, or enzyme, e.g. mild lysozyme). Resistant bacteria would be expected to arise from the binding interaction. This is an example of the use of non-homologous BRP/PRB pairs.

### Combination of fd and N

fd and IKe are similar filamentous phages with a overall homology of 55%. They recognise different BRPs (fd recognises the F' pilus, and IKe recognises pili encoded by N incompatibility group plasmids). However, their structure is sufficiently similar that the transfer of the PRB from one to the other should allow infection via the BRP recognised by the transferred PRB to occur.

It is known that antibodies can be placed within the PRB of fd. It would be expected that the insertion of antibodies (within the PRB of fd) which recognise the BRO encoded by the N incompatibility group plasmid would allow infection of that host. Furthermore, if the BRP (encoded by the N incompatibility group plasmid) was engineered to contain an epitope recognised by an antibody, infection should occur if that antibody was found within the PRB of fd. Manipulation of the BRP encoded by the N incompatibility group plasmids would have to be performed as described above for the BRP for fd.

This is another example of how the implementation of scheme 1 would allow the identification of a useful protein (the BRP encoded by the N incompatibility group plasmids).

### Alternative uses of Scheme 1

In addition to the selection of antibodies, or fragments thereof, from libraries of such molecules expressed on the surface of phage, this novel method may be used to select peptides or proteins which are able to bind other peptides or proteins. Although described for lamB, the examples described below could also be carried out with the fd/sex pilus, and fd/lamB systems, any other PRB/BRP combination, or any pair of proteins exposed on the surface of bacteria and phage as described in the "living column" as above.
1. Agonists or antagonists of growth factors, hormones, lymphokines or any other bioactive molecules. E.g. the expression of a receptor (or part thereof) for, or an antibody recognising, such a molecule within the lamB protein would allow selection of a binding protein or peptide from a random library of such molecules. The effect of the selected molecule on the biological system under study could then be tested.
2. Molecules capable of inhibiting the function of (pathogenic) viruses or bacteria. E.g. expression of CD4 (or part thereof), or the envelope protein (or part thereof) of HIV (the AIDS virus), within lamB would allow selection of a protein or peptide which may be able to inhibit the interaction of HIV with CD4 (its normal receptor).
3. Binding molecules, other than antibodies, useful in diagnostic procedures. E.g. the expression of tissue specific molecules within lamB would allow the selection of molecules which would have tissue specific binding by virtue of their selected phenotype.
4. Inhibitors or activators of the function of any protein whatsoever. E.g. the expression of an enzyme (or part thereof) within lamB would allow the selection of molecules which would be able to bind that enzyme. Further testing would reveal whether the molecues selected enhanced or inhibited the activity of the enzyme. Such effects would be expected to occur by the induction or stabilisation of conformational changes upon binding, by steric hindrance, by the inhibition of substrate binding or product release, or other mechanisms.
5. Novel enzymes with new or enhanced activities. The expression of reaction intermediate, or a molecule resembling such, within lamB would allow the selection of a protein capable of binding such a molecule. By careful choice of the form of the selected element (e.g. based on known canonical enzyme structures such as the TIM barrel, or catalytic antibodies), enzyme activities may be selected for.

As for phage antibodies, above, selected molecules may be used after they have cloned out of the J gene of lambda, or they may be used as lambda particles with specific binding properties or activities. In the former case they may be expressed as proteins and purified as such, or they may be expressed within cells, transgenic animals or plants, as individual proteins or as fusion proteins.

### Scheme 2: PRB as selector (Fig. 2)

The creation of antibody libraries will again be used to illustrate the methodology of scheme 2, bearing in mind that this scheme too, is applicable to any interaction between two protein species. Although the methods are described for lambda and lamB, any of the other phages described above could also be used.

The modified lambda DNA, carrying the J gene/peptide (antigen) fusion protein, is produced in a fashion similar to that described in scheme 1, replacing the ScFv described with DNA coding for the appropriate peptide or antigen. The DNA coding for the peptide is obtained by cloning, by PCR amplification using appropriate oligonucleotides, or by the use of oligonucleotides themselves to encode the desired amino-acid sequence. Appropriate sites have to be engineered into the ends of the oligonucleotides to allow cloning into appropriate sites. Best sites will be those not found, or found infrequently, within lambda. SpeI is such a site which can be incorporated at the 3' end of the J gene (and hence at the 5' end of the oligo encoding the peptide) without affecting J gene function. SacI is a site which can be used for the 3' end of the oligo encoding the peptide, as it is found within lambda in non-essential regions and so can be easily eliminated. A stop codon also has to be placed at the 3' end of the peptide sequence, if this is placed at the 3' end of the J gene.

Antibody variable region domains (or other protein species with binding activity) can be inserted within the coding region of lamB. In a usual antibody variable region domain comprising heavy and light chains, the N terminal ends of the heavy and light chains are far, in molecular terms, from their corresponding C terminals. As this may impose structural constraints upon the incorporation of variable region domains within the lamB protein, the removal of the first and last B pleated sheet from heavy and light chains should result in a compact variable region domain, the ends of which are very close together. This may help overcome the present size constraint of 70 amino-acids as the longest peptide successfully inserted into lamB.

It is envisaged that single VH domains or ScFvs can also be inserted into lamB. In the first case, soluble VL domains may also be expressed by the same cell into the periplasmic space to provide the necessary complement to create antigen binding activity.

A library of such molecules may be created in a fashion similar to that used for lambda phage described in scheme 1 above. The difference is that, instead of cloning into the J gene of lambda, the library of specificities would be cloned into the lamB gene within appropriately engineered sites in vectors AJC178, AJC264 or IC352. If soluble VL regions are used these would be encoded on a separate plasmid, or as a separate translation unit within the same plasmid. After transfection into e.g. pop6510, the library would be amplified and used for selection by phage.

In this scheme the interaction between phage and bacteria must result in survival of the bacteria to allow amplification of the element (ScFv, Fv or dAb in this example), selected by the selector found within the phage PRB (peptide antigen in this example). This can be done if a gene for a selectable marker (e.g. chloramphenicol, kanamycin resistance) is incorporated into the lambda vector under the control of a bacterial promoter. It is important that this resistance marker is different to that used for AJC264 (ampicillin), and found naturally within pop6510 (tetracycline), if this plasmid/bacterial strain combination is used. After infection, the growth of resistant bacterial colonies will be induced. A lysogenic strain of lambda, or one with a temperature sensitive mutation in cI (e.g. cI857), must be used in this system to prevent lysis of infected bacterial cells. In the latter case, colonies must be grown at 32°C, in order to preserve the activity of the lambda repressor and hence maintain lysogeny. The use of hfl (high frequency of lysogenisation) negative bacterial strains may increase the frequency of resistant colonies.

Bacterial colonies thus selected can be grown and manipulated in the normal fashion (albeit at lower temperature). The selected element can be identified following preparation and analysis (including DNA aquencing) of plasmid DNA. As for phage antibodies, above, selected molecules may be used after they have been cloned out of the lamB gene, or they may be used as bacterial particles with specific binding properties. In the former case they may be expressed as proteins and purified as such, or they may be expressed within cells, transgenic animals or plants, as individual proteins or as fusion proteins.

### Alternative uses of scheme 2

Scheme 2 may be used in exactly the same ways as scheme 1, a number of examples of which have been described above.

The decision whether to use scheme 1 or scheme 2 for a particular purpose will probably depend upon the nature of the selector and the selected element. It is expected that, in the J gene, for example, the size of inserted DNA sequences coding for selector or selected elements will be limited. The same is known to be true for lamB, however the experimental limits of the two systems have not yet been clearly defined. Given the apparent symmetry of schemes 1 and 2, it is clear that, all other things being equal, it would be advantageous to place the larger molecule (selector or selected element) within the protein (lamB or J) which has the greater "capacity". Another factor which should be taken into account is the stability of the fusion protein produced. Some proteins may be more stable in a system which involves a secretion step (lamB), whereas others may be more stable when expressed as part of a "cytoplasmic protein" - as lambda proteins can be considered to be, since they are synthesised intracellularly before the bacteria lyses to release the phage.

Another alternative reason for picking one system over another would be the final form of the selected element; i.e. selected elements expressed on phage may be more useful in some circumstances than if they were expressed on bacteria.

### Scheme 3: confrontation of libraries of specificities - the selection of couples (Fig. 3a)

In this scheme, two libraries of specificities, one on phage and one on bacteria (within the PRB and BRP, respectively) are confronted. Use of the terms selector and selected element is less valid in this case, as selection is not for a specific binding activity, but for an interaction which leads to infection of the bacteria by the phage. Instead, there are two libraries (A and B), individual elements of which (species A and species B) may be able to interact with one another. Within species A and species B, there are (for the purposes of the present invention at least) selectors and potential binding partners, and the terms "selector" and "binding partner" must each be understood to include a plurality of species.

Scheme 3a is operated in a fashion similar to that of scheme 2: i.e. the successful encounter of phage and bacteria will lead to bacterial colonies (rather than plaques) which will represent the productive binding of one element of species A with one of species B. As in scheme 2, the selection of colonies following interaction will depend upon the inclusion of a selectable marker within the lambda genome. Although the description below refers to lambda/lamB, any other BRP/PRB system could be used.

### Rescue of selected elements

The rescue of the selected element cloned into the lamB gene will occur in a fashion similar to that described above in scheme 2, i.e. growth and amplification of the selected bacterial colony and analysis of the plasmid DNA it contains, and the lambda it is able to produce.

The rescue of the selected element cloned into the J gene involves induction of the lysogenic bacterial colony. If the lambda vector contains a temperature sensitive mutant of the cI repressor (e.g. cI857), phage can be isolated from the colonies by heat induction (to inactivate the cI857 temperature sensitive repressor) or just by growth of the lysogenic bacteria, since phage are released naturally into the culture medium, even at lowered temperatures. The use of lowered temperatures may be important for the stability of the J/Ab fusion protein, as it is when antibodies (as ScFv or Fab) are grown in bacteria. The use of hfl bacterial strains may increase the frequency of amp resistance colonies; this may be important when screening a large number of possible binding interactions, and the use of lon protease negative bacteria may stabilise the fusion protein. Lambda, and DNA derived from it, can then be isolated from the supernatant using standard procedures.

As for schemes 1 and 2, above, selected molecules may be used after they have been cloned out of the lamB gene, or the J gene. They may be expressed as proteins and purified as such, or they may be expressed within cells, transgenic animals or plants, as individual proteins or as fusion proteins. Interacting pairs of molecules isolated in this way may also be studied in situ as phage/bacterial pairs, to determine, for example, affinity constants, interactions with other molecules and stability.

### Uses of scheme 3

The selection of an interacting pair rather than an individual selected element is likely to prove very useful under a number of different conditions. Antibody/antigen interaction is again used for the purpose of illustration.
1. The gene or cDNA coding for a protein of unknown function can be used to produce a monoclonal phage (or bacterial) antibody, using schemes 1 or 2 above. However, by the use of scheme 3, this powerful technique can be extended. Instead of the use of a single selector to isolate the antibody, a library of fragments derived from the cDNA or gene can be used. These can be isolated after appropriate restriction digestion or PCR amplification. After library confrontation, phage (or bacterial) antibodies binding to different parts of the cDNA or gene used will be produced. This will yield not only a "polyclonal phage (or bacterial) antibody" directed against different epitopes which can be used as a mixture, but also a rich source of monoclonal phage (or bacterial) antibodies which can be used individually.
2. The study of structure-function relationships between antigen/antibody, or indeed any other protein/protein interaction is greatly facilitated. An antibody against a protein antigen X may be randomly mutated, to produce a library of anti-X antibodies. This library then may be confronted with a similar library of mutants of the antigen X. Mutations in either antibody or antigen which inhibited the normal interaction of anti-X with X, could only be selected for by the binding to partners with compensatory mutations. In this way one may select pairs of anti-X/X which were able to bind to one another but not to the unmutated partner. By the sequencing of such pairs one would obtain a greater understanding of the molecular nature of the interaction of X with anti-X, without the need for X ray crystallography. Examples of other protein interactions which could be studied using this method include: transcriptional activator/repressor pairs, proteins involved in the uptake of viruses by cells (e.g. CD4, HIV envelope protein), individual elements in multi-protein or enzyme complexes.
3. The creation of "polyclonal phage (or bacterial) antibodies" against the protein product of a cDNA or gene described above can be extended to allow the selection of phage or bacterial antibodies against any coding sequence at random. This is likely to prove very useful and important in situations in which large amounts of DNA sequence data are acquired at random. This is the case in the projects underway to sequence a number of different genomes (such as E. coli, yeast, C. elegans, Drosophila, mouse and human). The identification of potential coding sequences can be undertaken using computer analysis or exon trapping via retroviral intermediates; these can then be isolated en masse using PCR amplification with appropriate oligonucleotides. A library of such random coding sequences can be used to select from a library of random antibodies (or indeed any binding proteins) those which bind. The isolation of bacteria/phage pairs will give, in one step, antibodies against the protein products of defined DNA sequences.
4. The accumulation and analysis of DNA sequence data has revealed that many proteins have counterparts encoded by similar genes. In many cases the assignment of the gene encoding a particular protein to a family of genes is made on the basis of the conservation of certain sequence motifs (e.g. immunoglobulin superfamily, family of tyrosine kinases, family of DNA binding proteins such as homeodomains or zinc finger-like domains)) . This conservation of sequence motif has been used to isolate other members of the same family by either standard library screening using DNA hybridisation, or by PCR amplification using the conserved sequences to design the amplifying oligonucleotides employed. It is clear that this use of PCR amplification of related genes can be used in scheme 3. Such related genes can be cloned into the lamB gene using oligonucletides corresponding to conserved regions of these gene families. This will create a library of coding fragments of related genes which can be used to derive phage antibodies, or binding peptides, which will have a range of specificities, from specific for individual members of the gene family, to general specificities which recognise all members of the family.
5. If both libraries A and B consist of antibodies, the interactions which will be selected for will be of the nature: idiotype/anti-idiotype. The isolation of a large number of these will prove to be useful in immunological studies as well as in future antibody engineering.

In cases 2, 3 and 4 above, the likelihood that useful antibodies are selected can be increased by using the library of bacteria (expressing the fragments of coding sequences within lamB) to immunise mice, and then to use the spleen of such mice to create the library of antibody specificities.

It is important to note that in scheme 3 confrontations of libraries can occur with no knowledge of the precise DNA sequence of the elements A and B. This will occur, for example, if mutations (created at random) in known sequences are used to create the libraries, or unknown sequences derived following PCR amplification. The precise sequence of the interacting elements will not be known until after they have interacted, and then been isolated and analysed.

### Scheme 3b: confrontation of libraries of specificities - the selection of singles (Fig. 3b)

An alternative to the selection of couples of interacting elements, as described above, is the selection of single non-interacting lements from a pool of interacting ones. Also in this scheme two libraries of specificities, one on bacteria and one on phage, will be confronted. Although, in principle, the libraries do not need to a restricted or preselected to allow selection of singles, it is clear that to fully exploit this scheme the two libraries should be reselected, according to any of the schemes above. This is because non-selected libraries will contain many spurious singles. Preselection of libraries will ensure that the selection of singles occurs from within a population with a predetermined pattern of specificities.

### Rescue of selected singles

Two cases are to be distinguished: the selection of phage not interacting with any elements in the library expressed within the BRP, and the selection of bacteria not interacting with any elements in the library expressed within the PRB.

### Selection of bacterial singles

To select non-interacting bacteria from a library of bacteria expressing different specificities within their BRP upon challenge with a library of phage expressing different specificities within their PRB, it is only necessary to perform the confrontation under lytic conditions. All bacteria which can be recognised by phage will be lysed, and the only ones resistant to lysis will be those expressing a specificity not recognised by an element within the phage library. Surviving bacteria can be amplified and rescued.

### Selection of phage singles

There are two ways in which non-interacting phage from a library of phage expressing different specificities within their PRB upon challenge with a library of bacteria expressing different specificities within their BRP can be selected:
a) Absorption of the phage library upon the bacterial library under condition in which infection is prevented.
b) Infection of the bacterial library with the phage library under strictly lysogenic conditions. Interacting phage will disappear as phage particle as they are taken up by binding bacteria.

Phage particles will be able to be rescued and amplified using standard methods from a supernatant in both cases.

### Application of scheme 3b in the analysis of human genetic disease

The use of scheme 3b to derive specific probes (DNA, antibody or binding proteins or peptides) for modifications at genetic loci, such as those found in many genetic diseases, is readily envisaged. This will involve the combination of scheme 3b with schemes 1 and 2 above. An example ia the identification by RFLP, or other genetic methods, of the linkage between a certain human genetic disease and a region of DNA. The resolution of genetic techniques is usually of the order of hundreds of kilobases. The cloning of a library of coding regions from this chromosomal locus from both normal (termed the N epitope library) and mutated (termed the M epitope library) forms of the locus into lamB could provide libraries of epitopes from which one may select:
a) specific phage antibodies to the unknown mutated epitopes.
b) specific phage antibodies to the normal versions of the mutated epitopes.
c) candidates for the unknown mutated epitope, or
d) in the case of mutations which involve deletions, candidates for the normal epitope lost or affected by the disease mutation.

By way of example, after challenge of the N epitope library with a phage antibody library (either general or derived following immunisation with bacteria expressing the N epitope library) using scheme 3, infectious phage antibodies (termed the N restricted phage antibody library) harvested may be used to:
a) Infect the M epitope library under conditions allowing bacterial lysis. Surviving bacteria carry epitopes not recognised by the N restricted phage antibody library and therefore potential candidates for (or an epitope of) the mutated locus.
b) Absorb the N restricted phage antibody library on an excess of the M epitope library (under conditions where infection will not occur). Phages failing to be absorbed will be candidate phage antibodies for the normal version of the mutated epitope.
c) An alternative to the use of absorption to derive candidate phage antibodies for the normal version of the mutated epitope is to perform the challenge of the N phage antibody library with the M epitope library under stringent lysogenic conditions. Under these conditions, the only phage surviving as phage particles will be candidates for those recognising the normal version of the mutated locus.

The scheme can and should be reversed using M phage antibody libraries (derived by challenging the M epitope library with a phage antibody library). This M phage antibody library could then used to:
a) Infect the N epitope library under conditions allowing bacterial lysis. Surviving bacteria would carry epitopes not recognised by the M phage antibody library and would therefore be potential candidates for (or an epitope of) the normal version of the mutated locus.
b) Absorb the M phage antibody library on an excess of the N epitope ibrary (under conditions where infection will not occur). Phages failing to be absorbed will be candidate phage antibodies for the mutated epitope.
c) Challenge the M phage antibody library with the N epitope library under stringent lysogenic conditions. Under these conditions the only phage surviving as phage particles will be candidates for those recognising the mutated locus.

## Claims

1. A method for selecting a binding partner for a selector protein from among a library of proteins expressed on the surface of a first transformed microorganism, which comprises screening the library with a second microorganism transformed such that it expresses the selector protein on its surface, wherein one of the first and second microorganisms is a bacterium and the other is a bacteriophage.

2. A method according to claim 1, wherein one of the library and the selector protein is expressed within the phage receptor for bacteria (PRB) and the other is expressed within the bacterial receptor for phage (BRP), the microorganisms being transformed such that interaction between PRB and BRP occurs only if the binding partner and the selector protein are able to interact.

3. A method according to claim 2, wherein the phage lyses the bacterium.

4. A method according to claim 2, whereby the bacterium acquires a resistance phenotype after interaction with the phage.

5. A method according to claim 2, whereby the bacterium expresses a new gene after interaction with the phage.

6. A method according to claim 3, which additionally comprises selecting and amplifying the lytic phage.

7. A method according to claim 4 or claim 5, which additionally comprises selecting and amplifying the lysogenic, resistant or new gene-expressing bacterium.

8. A method according to any preceding claim, wherein the selector protein is one of a library expressed by the second microorganism.

9. A method according to claim 8, wherein respective libraries are expressed within the phage receptor for bacteria and within the bacterial receptor for phage, and which comprises the additional step of amplifying selected phage/bacterial partners.

10. A method according to claim 8, wherein respective libraries are expressed within the phage receptor for bacteria and within the bacterial receptor for phage, and which comprises the additional step of amplifying non-interacting phage or bacteria.

11. A method according to any preceding claim, wherein the bacterium and phage are respectively E. coli and bacteriophage λ.

12. A method according to claim 11, wherein the bacteriophage λ expresses antibody within its J gene protein, and the E. coli expresses antigen within its lamB protein.

13. A method according to any of claims 1 to 10, wherein the bacterium includes a F' pilus and the phage is fd (M13,f1) phage.

14. A method according to any of claims 1 to 10, wherein the phage, the bacterium and the respective receptors are selected from T4 gene 37 protein/omp C and T2 gene 38 protein/omp F.

15. A method according to any preceding claim, for the selection of agonists or antagonists of growth factors, hormones, lymphokines or other bioactive molecules, or receptors, or parts thereof.

16. A method according to any preceding claim, for the selection of molecules capable of inhibiting the function of pathogenic viruses or bacteria.

17. A method according to any preceding claim, for the selection of binding molecules useful in diagnostic procedures.

18. A method according to any preceding claim, for the selection of inhibitors or activators of the function of the protein.

19. A method according to claim 18, wherein said protein is an enzyme.

20. A method according to any of claims 1 to 14, for the selection of novel enzymes with respect to a reaction intermediate.

21. A method according to any of claims 1 to 14, for the selection of a monoclonal antibody produced in bacterium or phage.

22. A method according to claim 21, for the selection of bacterial or phage antibodies against the protein products of a given coding sequence or library of coding sequences.

23. A method according to any of claims 1 to 14, for the selection of phage antibodies or binding proteins recognising the protein products of the members of a gene family specified by one or more conserved motifs.

24. A method according to any of claims 1 to 14, for the selection of anti-idiotypic antibodies.

25. A method according to any of claims 1 to 14, for the independent selection of first and second binding partners respectively corresponding to chromosomal regions of normal subjects and of those carrying a genetic disease, and which additionally comprises comparing the binding partners.

26. A method according to claim 23, for obtaining (a) phage antibodies or other binding molecules to the gene mutations, or clones therefor, or (b) phage antibodies or other binding molecules to the normal counterparts of the mutations, or clones therefor.

27. A method according to any preceding claim, for the selection of interactions between first and second mutated proteins which interacted before mutation but not thereafter.

28. A method according to any preceding claim, wherein the partner-selector protein binding protects the transformed microorganism from death caused by, for example, phage binding.

29. A method according to any preceding claim, wherein one of the transformed microorganism is immobilised on a solid phase.

## Patentansprüche

1. Verfahren zur Auswahl eines Bindungspartners für ein Selektorprotein aus einer Bank von Proteinen, die auf der Oberfläche eines ersten transformierten Mikroorganismus exprimiert werden, welches umfaßt das Screenen der Bank mit einem zweiten Mikroorganismus, der so transformiert ist, daß er das Selektorprotein auf seiner Oberfläche exprimiert, wobei einer der ersten und zweiten Mikroorganismen ein Bakterium und der andere ein Bakteriophage ist.

2. Verfahren nach Anspruch 1, worin das eine von Bank und Selektorprotein im Rahmen des Phagenrezeptors für Bakterien (PRB) und das andere im Rahmen des bakteriellen Rezeptors für Phagen (BRP) exprimiert wird, wobei die Mikroorganismen so transformiert sind, daß eine Wechselwirkung zwischen PRB und BRP nur stattfindet, wenn der Bindungspartner und das Selektorprotein in der Lage sind, miteinander zu wechselwirken.

3. Verfahren nach Anspruch 2, worin der Phage das Bakterium lysiert.

4. Verfahren nach Anspruch 2, worin das Bakterium nach der Wechselwirkung mit dem Phagen einen Resistenz-Phänotyp erwirbt.

5. Verfahren nach Anspruch 2, worin das Bakterium nach der Wechselwirkung mit dem Phagen ein neues Gen exprimiert.

6. Verfahren nach Anspruch 3, welches ferner die Selektion und Amplifizierung des lytischen Phagen umfaßt.

7. Verfahren nach Anspruch 4 oder Anspruch 5, welches ferner die Selektion und Amplifizierung des lysogenen, resistenten oder ein neues Gen exprimierenden Bakteriums umfaßt.

8. Verfahren nach irgendeinem der vorhergehenden Ansprüche, worin das Selektorprotein eines aus einer Bank ist, die von dem zweiten Mikroorganismus exprimiert wird.

9. Verfahren nach Anspruch 8, worin die jeweiligen Banken im Rahmen des Phagenrezeptors für Bakterien und im Rahmen des bakteriellen Rezeptors für Phagen exprimiert werden und welches den weiteren Schritt der Amplifizierung ausgewählter Phagen/Bakterien-Partner umfaßt.

10. Verfahren nach Anspruch 8, worin die jeweiligen Banken im Rahmen des Phagenrezeptors für Bakterien und im Rahmen des bakteriellen Rezeptors für Phagen exprimiert werden und welches den weiteren Schritt der Amplifizierung nicht wechselwirkender Phagen oder Bakterien umfaßt.

11. Verfahren nach irgendeinem der vorhergehenden Ansprüche, worin das Bakterium und der Phage E. coli bzw. Bakteriophage λ sind.

12. Verfahren nach Anspruch 11, worin der Bakteriophage λ Antikörper im Rahmen seines J-Gen-Proteins exprimiert und das E. coli Antigen im Rahmen seines lamß-Proteins exprimiert.

13. Verfahren nach irgendeinem der Ansprüche 1 bis 10, worin das Bakterium einen F'-Pilus einschließt und der Phage ein fd (M13, f1) -Phage ist.

14. Verfahren nach irgendeinem der Ansprüche 1 bis 10, worin der Phage, das Bakterium und die jeweiligen Rezeptoren aus T4-Gen 37-Protein/omp C und T2-Gen 38-Protein/omp F ausgewählt sind.

15. Verfahren nach irgendeinem der vorhergehenden Ansprüche zur Selektion von Agonisten oder Antagonisten von Wachstumsfaktoren, Hormonen, Lymphokinen oder anderen bioaktiven Molekülen oder Rezeptoren oder Teilen davon.

16. Verfahren nach irgendeinem der vorhergehenden Ansprüche zur Selektion von Molekülen, welche zur Inhibierung der Funktion von pathogenen Viren oder Bakterien in der Lage sind.

17. Verfahren nach irgendeinem der vorhergehenden Ansprüche zur Selektion von bindenden Molekülen, die für diagnostische Verfahren geeignet sind.

18. Verfahren nach irgendeinem der vorhergehenden Ansprüche zur Selektion von Inhibitoren oder Aktivatoren der Funktion des Proteins.

19. Verfahren nach Anspruch 18, worin das Protein ein Enzym ist.

20. Verfahren nach irgendeinem der Ansprüche 1 bis 14 zur Selektion neuer Enzyme mit Bezug zu einem Reaktionszwischenprodukt.

21. Verfahren nach irgendeinem der Ansprüche 1 bis 14 zur Selektion eines monoklonalen Antikörpers, der in einem Bakterium oder Phagen erzeugt wird.

22. Verfahren nach Anspruch 21 zur Selektion von bakteriellen oder Phagen-Antikörpern gegen die Proteinprodukte einer gegebenen kodierenden Sequenz oder Bank von kodierenden Sequenzen.

23. Verfahren nach irgendeinem der Ansprüche 1 bis 14 zur Selektion von Phagen-Antikörpern oder bindenden Proteinen, welche die Proteinprodukte der Mitglieder einer Genfamilie erkennen, die durch ein oder mehrere konservierte(s) Motiv(e) gekennzeichnet ist.

24. Verfahren nach irgendeinem der Ansprüche 1 bis 14 zur Selektion von anti-idiotypischen Antikörpern.

25. Verfahren nach irgendeinem der Ansprüche 1 bis 14 zur unabhängigen Selektion von ersten und zweiten Bindungspartnern, die chromosomalen Bereichen von normalen Personen bzw. denjenigen, die eine genetische Krankheit tragen, entsprechen, welches ferner den Vergleich der Bindungspartner umfaßt.

26. Verfahren nach Anspruch 23 zur Gewinnung von (a) Phagen-Antikörpern oder anderen bindenden Molekülen für die Genmutationen oder von Klonen dafür, oder (b) Phagen-Antikörpern oder anderen bindenden Molekülen für die normalen Gegenstücke der Mutationen oder von Klonen dafür.

27. Verfahren nach irgendeinem der vorhergehenden Ansprüche zur Selektion von Wechselwirkungen zwischen ersten und zweiten mutierten Proteinen, welche vor der Mutation miteinander wechselwirkten, jedoch nicht nachher.

28. Verfahren nach irgendeinem der vorhergehenden Ansprüche, worin die Partner-Selektorprotein-Bindung den transformierten Mikroorganismus vor einem Tod schützt, der beispielsweise durch Phagenbindung verursacht wird.

29. Verfahren nach irgendeinem der vorhergehenden Ansprüche, worin einer der transformierten Mikroorganismen auf einer festen Phase immobilisiert ist.

## Revendications

1. Procédé pour sélectionner un partenaire de liaison pour une protéine sélectrice dane une bibliothèque de protéines exprimées à la surface d'un premier micro-organisme transformé, qui comporte l'examen de la bibliothèque par un deuxième micro-organisme transformé de manière à exprimer la protéine sélectrice à sa surface, dans lequel l'un parmi le premier et le deuxième micro-organismes est une bactérie, et l'autre est un bactériophage.

2. Procédé selon la revendication 1, dans lequel l'une parmi la protéine de la bibliothèque et la protéine sélectrice est exprimée à l'intérieur du récepteur du phage pour la bactérie (PRB), et l'autre est exprimée à l'intérieur du récepteur de la bactérie pour le phage (BRP), les micro-organismes étant transformés de telle sorte que l'interaction entre le PRB et le BRP ait lieu uniquement si le partenaire de liaison et la protéine sélectrice sont capables d'interagir.

3. Procédé selon la revendication 2, dans lequel le phage lyse la bactérie.

4. Procédé selon la revendication 2, dans lequel la bactérie acquiert un phénotype de résistance après l'interaction avec le phage.

5. Procédé selon revendication 2, dans lequel la bactérie exprime un nouveau gène après interaction avec le phage.

6. Procédé selon la revendication 3, qui comporte en supplément la sélection et l'amplification du phage lytique.

7. Procédé selon la revendication 4 ou la revendication 5, qui comporte en supplément la sélection et l'amplification de la bactérie lysogène, résistante ou exprimant un nouveau gène.

8. Procédé selon l'une quelconque des revendications précédentes, dans lequel la protéine sélectrice est une protéine d'une bibliothèque exprimée par le deuxième micro-organisme.

9. Procédé selon la revendication 8, dans lequel des bibliothèques respectives sont exprimées à l'intérieur du récepteur du phage pour la bactérie et à l'intérieur du récepteur de la bactérie pour le phage, et qui comporte l'étape supplémentaire d'amplification de partenaires phage/bactérie sélectionnée.

10. Procédé sera la revendication 8, dans lequel des bibliothèques respectives sont exprimées à l'intérieur du récepteur du phage pour la bactérie et à l'intérieur du récepteur de la bactérie pour le phage, et qui comporte l'étape supplémentaire d'amplification du phage ou de bactéries n'entrant pas en interaction.

11. Procédé selon l'une quelconque des revendications précédentes, dans lequel la bactérie et le phage sont respectivement E. coli et le bactériophage λ.

12. Procédé selon la revendication 11, dans lequel le bactériophage λ exprime un anticorps dans sa protéine de gène J, et E. coli exprime un antigène dane sa protéine lamβ.

13. Procédé selon l'une quelconque des revendications 1 à 10, dans lequel la bactérie comporte un pilus F' et le phage est un phage fd (M13,f1).

14. Procédé selon l'une quelconque des revendications 1 à 10, dans lequel le phage, la bactérie et les récepteurs respectifs sont sélectionnés parmi la protéine/omp C du gène 37 de T4 et la protéine/omp F ou gène 38 de T2.

15. Procédé selon l'une quelconque des revendications précédentes, pour la sélection d'agonistes ou d'antagonistes de facteurs de croissance, d'hormones, de lymphokines ou d'autres molécules bioactives, ou de récepteurs ou de parties de ces derniers.

16. Procédé selon l'une quelconque des revendications précédentes, pour la sélection de molécules capables d'inhiber la fonction de bactéries ou de virus pathogènes.

17. Procédé selon l'une quelconque des revendications précédentes, pour la sélection de molécules de liaison utiles dans des modes opératoires de diagnostic.

18. Procédé selon l'une quelconque des revendications précédentes, pour la sélection d'inhibiteurs ou d'activateurs de la fonction de la protéine.

19. Procédé selon la revendication 18, dans lequel ladite protéine est une enzyme.

20. Procédé selon l'une quelconque des revendications 1 à 14, pour la sélection de nouvelles enzymes vis-à-vis d'un intermédiaire de réaction.

21. Procédé selon l'une quelconque des revendications 1 à 14, pour la sélection d'un anticorps monoclonal produit dans une bactérie ou un phage.

22. Procédé selon la revendication 21, pour la sélection d'anticorps de bactéries ou de phages dirigés contre les protéines produites par une séquence codante donnée ou une bibliothèque de séquences codantes.

23. Procédé selon l'une quelconque des revendications 1 à 14, pour la sélection d'anticorps de phages ou de protéines de liaison reconnaissant les protéines produites par les membres d'une famille de gènes spécifiée par un ou plusieurs motifs conservés.

24. Procédé selon l'une quelconque des revendications 1 à 14, pour la sélection d'anticorps anti-idiotypiques.

25. Procédé selon l'une quelconque des revendications 1 à 14, pour la sélection indépendante de premiers et de deuxièmes partenaires de liaison correspondant respectivement à des régions chromosomiques de sujets normaux et de sujets porteurs d'une maladie génétique, et qui comporte en supplément la comparaison des partenaires de liaison.

26. Procédé selon la revendication 23, pour obtenir (a) des anticorps de phages ou d'autres molécules de liaison vis-à-vis des mutations génétiques, ou de clones de celui-ci, ou (b) des anticorps de phages ou d'autres molécules de liaison aux complémentaires normaux des mutations ou à des clones de ceux-ci.

27. Procédé selon l'une quelconque des revendications précédentes, pour la sélection d'interactions entre des premières et des secondes protéines mutées qui interagissent avant mutation mais pas après.

28. Procédé selon l'une quelconque des revendications précédentes, dans lequel la liaison entre le partenaire et la protéine sélectrice protège le micro-organisme transformé de la mort provoquée par exemple par une liaison à un phage.

29. Procédé selon l'une quelconque des revendications précédentes, dans lequel l'un des micro-organismes transformés est immobilisé sur une phase solide.
